Europäisches Patentamt

European Patent Office

Office européen des brevets

(19)

(11) **EP 0 796 254 B1**

(12) **FASCICULE DE BREVET EUROPEEN**

(45) Date de publication et mention
de la délivrance du brevet:
**18.04.2001 Bulletin 2001/16**

(21) Numéro de dépôt: **95941777.5**

(22) Date de dépôt: **06.12.1995**

(51) Int Cl.[7]: **C07D 307/46**

(86) Numéro de dépôt international:
**PCT/FR95/01612**

(87) Numéro de publication internationale:
**WO 96/17836 (13.06.1996 Gazette 1996/27)**

(54) **PROCEDE DE FABRICATION SELECTIVE DE FURANEDICARBOXALDEHYDE-2,5 A PARTIR DE L'HYDROXYMETHYL-5 FURANE CARBOXALDEHYDE-2**

VERFAHREN ZUR SELEKTIVEN HERSTELLUNG VON 2,5-FURANDICARBOXAL-DEHYD AUS 5-HYDROXYLMETHYL-2-FURANCARBOXALDEHYD

METHOD FOR SELECTIVELY PREPARING 2,5-FURANDICARBOXALDEHYDE FROM 5-HYDROXYMETHYL FURAN 2-CARBOXALDEHYDE

(84) Etats contractants désignés:
**AT BE CH DE DK ES FR GB IT LI LU NL PT**

(30) Priorité: **07.12.1994 FR 9414958**

(43) Date de publication de la demande:
**24.09.1997 Bulletin 1997/39**

(73) Titulaire: **AGRICHIMIE**
**F-97231 Robert (FR)**

(72) Inventeurs:
• **DURAND, Germain**
**F-34830 Clapiers (FR)**
• **FAUGERAS, Pierre**
**F-30130 Pont-Saint-Esprit (FR)**
• **LAPORTE, Françoise**
**F-30165 Saint-Laurent-des-Arbres (FR)**
• **MOREAU, Claude**
**F-34880 Laverune (FR)**
• **NEAU, Marie-Claude**
**F-31290 Gardouch (FR)**
• **ROUX, Gabriel**
**F-38240 Meylan (FR)**

• **TICHIT, Didier, Rés. Le Vallon-des-Sources,**
**F-34090 Montpellier (FR)**
• **TOUTREMEPUICH, Cécile**
**F-06400 Cannes (FR)**

(74) Mandataire:
**Cabinet BARRE LAFORGUE & associés**
**95, rue des Amidonniers**
**31000 Toulouse (FR)**

(56) Documents cités:
**EP-A- 0 356 703          FR-A- 2 669 636**

• **CHEMICAL ABSTRACTS, vol. 108, no. 3, 18 Janvier 1988 Columbus, Ohio, US; abstract no. 21707, IOVEL I. G. 'Preparation of 2,5-furandicarboxaldehyde' & SU,A,1 342 903 (INSTITUTE OF ORGANIC SYNTHESIS, ACADEMY OF SCIENCES, LATVIAN S.S.R.)**

Remarques:
Le dossier contient des informations techniques présentées postérieurement au dépôt de la demande et ne figurant pas dans le présent fascicule.

# EP 0 796 254 B1

## Description

L'invention concerne un procédé de fabrication sélective du furanedicarboxaldéhyde-2,5 (FDC) à partir de l'hydroxyméthyl-5 furane carboxaldéhyde-2 (HMF).

Le HMF peut être synthétisé à partir de substances d'origine végétale contenant ou pouvant libérer du fructose ou des polyfructanes, par exemple le jus de topinambour. Le HMF constitue un produit de départ dans la synthèse de nombreux dérivés furaniques qui, à leur tour peuvent servir dans la production de polymères qui présentent des propriétés intéressantes (possibilité de modifications chimiques particulières, tenue en température et au feu, inertie chimique, propriétés mécaniques et/ou électriques particulières...).

Parmi les nombreux dérivés du HMF, le FDC est un monomère qui peut faire l'objet de nombreuses applications. En particulier, il peut servir : pour la synthèse de certains polymères et de macrocycles, notamment dans le domaine pharmaceutique ; d'agent de réticulation dans la préparation de polymères spéciaux ; de liant dans les sables de fonderie ; pour la fabrication de la pellicule de séparation des solutions aqueuses des batteries alcalines ; d'agent d'inhibition de corrosion ; d'agent de traitement de surface de métaux tels que le cuivre, le nickel ; d'intermédiaire de synthèse d'autres composés furaniques symétriques ou dissymétriques tels que l'acide furane-2-5-dicarboxylique (FDA), le furane 2-5-diméthylamine...

Le FDC n'est cependant pas fabriqué industriellement en raison de l'absence de procédé économiquement rentable pouvant être mis en oeuvre en continu et dans des conditions compatibles avec les exigences actuelles en matière de respect de l'environnement.

Les procédés connus de synthèse du FDC sont soit des réactions stoechiométriques (qui présentent les inconvénients d'être très polluantes, d'engendrer des problèmes de corrosion des installations, d'être coûteuses et difficilement réalisables en continu), soit des réactions en catalyse homogène (qui sont difficilement réalisables en continu et nécessitent une séparation des produits de réaction dissous).

Les réactions d'oxydation du HMF en catalyse hétérogène posent a priori des difficultés dans la mesure où le HMF n'est pas stable aux hautes températures (sa dégradation intervenant à partir de 100° C) nécessaires à l'activation des catalyseurs solides d'oxydation.

L'oxydation du HMF en milieu liquide en catalyse hétérogène a cependant déjà été décrite. Par exemple, la demande de brevet DE-A-3.826.073 préconise l'emploi de platine sur charbon actif à titre de catalyseur à une température de 70° C pour oxyder une solution aqueuse de HMF. La réaction n'est pas sélective en FDC (le rendement en FDC étant de 24 %) et produit aussi de l'acide formyl-5 furane carboxylique-2 (FFCA) et de l'acide furane dicarboxylique-2,5. Egalement, la publication "Platinium Catalyzed Oxidation of 5-hydroxymethylfurfural" P. VINKE, H.E. van DAM, H. van BEKKUM, New Developments in Selective Oxidation, G. GENTI et F. TRIFIRO Editeur, Elsevier Science Publishers, Amsterdam, Studies in Surface Science and Catalysis, 1990, 55, p 147-156, décrit une réaction d'oxydation du HMF en milieu aqueux catalysée par du platine sur alumine à 60° C en présence d'oxygène. La réaction produit très peu de FDC et est surtout indiquée pour fabriquer sélectivement le FFCA.

Par ailleurs, de nombreuses synthèses d'aldéhydes à partir d'alcools en catalyse hétérogène sont connues de l'art antérieur. Une multitude de catalyseurs ont été utilisés dans ces diverses réactions : la plupart des oxydes métalliques bruts ou supportés (fer, cuivre, zinc, argent, nickel, cobalt, magnésium, vanadium, zirconium, molybdène, bismuth, antimoine...), la silice, les silicates, les zéolithes... Dans la plupart des cas, ces réactions sont effectuées en phase gazeuse à haute température (plus de 200° C). Ainsi, le document CHEMICAL ABSTRACTS, vol. 108, n° 3, 18 janvier 1988, abstract n° 21707, mentionne un procédé de préparation de FDC par oxydation de 2,5-diméthylfurane ou de 5-méthylfurfural en phases vapeur et gazeuse, en présence d'oxygène atmosphérique et à une température de 250 à 475° C en présence de $V_2O_5$ ou d'un mélange de 15 à 60 % de $V_2O_5$ et 85 à 40 % de Mo O3 supporté sur corindon. Toutes ces réactions ne peuvent donc pas a priori être transposées à l'oxydation du HMF du fait de son instabilité thermique qui impose que la réaction se déroule en phase liquide et à basse température (moins de 200° C).

Dans toute la présente demande, on utilise les termes de sélectivité, de conversion et de rendement en référence aux définitions suivantes :

$$C\ (\%\ molaire) = \frac{quantité\ de\ HMF\ transformée}{quantité\ de\ HMF\ initiale} \times 100$$

$$S\ (\%\ molaire) = \frac{quantité\ de\ FDC\ formée}{quantité\ de\ HMF\ transformée} \times 100$$

$$R\ (\%\ molaire) = S \times C/100 = \frac{quantité\ de\ FDC\ formée}{quantité\ de\ HMF\ initiale} \times 100$$

avec :

2

C = conversion,
S = sélectivité,
R = rendement.

Dans ce contexte, la présente, invention vise un procédé de fabrication sélective du FDC à partir du HMF. L'invention vise en particulier un tel procédé qui présente des qualités autorisant son exploitation industrielle (peu polluant, facile à mettre en oeuvre en continu, rapide, peu coûteux et de rendement satisfaisant). A cet égard, il est à noter que tant la sélectivité que la conversion du procédé sont essentielles afin de faciliter, ou même d'éviter, les étapes subséquentes, généralement coûteuses, de séparation ou de purification.

Ainsi, l'invention vise en particulier à proposer un procédé dont le rendement en FDC est supérieur à 60 %, et notamment dont la sélectivité en FDC et la conversion sont toutes deux supérieures à 90 %.

A cet effet, l'invention concerne un procédé de fabrication sélective du FDC à partir du HMF en phase liquide et à basse température (moins de 200° C).

Dans un procédé selon l'invention, on met le HMF dissous dans un solvant liquide au contact d'au moins un composé solide comprenant au moins un métal choisi parmi les métaux des colonnes IV B, V B et VI B de la classification périodique des éléments, et on place le milieu réactionnel à une température comprise entre 75° C et 200° C.

Parmi les métaux pouvant être utilisés, on peut citer en particulier le titane, le zirconium, le vanadium, le niobium, le chrome, le molybdène et le tungstène. Ces métaux peuvent être utilisés sous forme métallique pure ou de sel, ou de préférence sous forme d'oxydes simples ou mixtes. En outre, ces métaux peuvent être utilisés à l'état massique (non supportés) ou au contraire sur un support. Dans ce dernier cas, le support peut aussi incorporer un métal ou être formé d'un silicate, notamment un tectosilicate ou une argile.

Avantageusement, on utilise au moins un composé solide comprenant de l'oxyde de vanadium. En effet, des résultats nettement améliorés ont été obtenus, de façon surprenante, avec l'oxyde de vanadium $V_2O_5$.

Selon l'invention, on effectue la réaction en présence d'oxygène, notamment dans un réacteur dans lequel on maintient une pression de gaz comprenant de l'oxygène. Avantageusement et selon l'invention, on effectue la réaction dans un réacteur dans lequel on maintient une pression d'air comprise entre $5.10^5$ Pa et $30.10^5$ Pa. L'oxygène permet de régénérer en permanence l'oxyde de vanadium qui fait office simultanément d'oxydant et de catalyseur solide. Plus précisément, on maintient une pression partielle d'oxygène suffisante pour maintenir l'oxyde de vanadium à un niveau d'oxydation optimum pour la réaction.

Le composé solide peut être constitué d'oxyde de vanadium à l'état libre non supporté (c'est-à-dire massique). Dans ce cas, la réaction est de préférence effectuée à une température supérieure à 150° C, notamment de l'ordre de 170° C.

Néanmoins, avantageusement et selon l'invention, on utilise, à titre de composé solide, de l'oxyde de vanadium supporté sur un support d'oxyde métallique de type amphotère à prédominance acide. On a en effet pu mettre en évidence un effet de synergie entre l'oxyde de vanadium et son support d'oxyde métallique, notamment lorsque ce dernier n'est ni acide, ni basique, ni amphotère à prédominance basique. En particulier et selon l'invention, on utilise de l'oxyde de vanadium supporté sur un support d'oxyde de titane. Avantageusement et selon l'invention, le support d'oxyde de titane est formé d'oxyde de titane comprenant entre 50 % et 100 % de phase anatase et entre 50 % et 0 % de phase rutile, plus particulièrement entre 60 % et 75 % de phase anatase et entre 40 % et 25 % de phase rutile, notamment de l'ordre de 64 % de phase anatase et de l'ordre de 36 % de phase rutile. Dans ces conditions en effet, on peut effectuer la réaction à une température très basse, inférieure à 100° C, notamment de l'ordre de 90° C, tout en obtenant une conversion et une sélectivité toutes deux supérieures à 90 %.

Les performances du procédé selon l'invention dépendent également de la proportion pondérale du vanadium dans le composé solide. Avantageusement et selon l'invention, on utilise un composé solide comprenant entre 1 % et 56 %, plus particulièrement plus de 4 %, en poids de vanadium. De bons résultats ont été obtenus pour une proportion pondérale du vanadium dans le composé solide comprise entre 7 % et 7,5 %, notamment de l'ordre de 7,33 %.

En outre, on utilise le composé solide selon un rapport massique par rapport à la quantité initiale de HMF compris entre 0,5 et 5, notamment de l'ordre de 2.

Selon l'invention, on place le HMF dans un solvant polaire liquide apte à solubiliser le HMF et à rester chimiquement inerte à la température de réaction et vis-à-vis du composé solide. Selon l'invention, le solvant polaire doit également être apte à solubiliser le FDC. Selon l'invention, le solvant est avantageusement un solvant aromatique ou une cétone.

Avantageusement et selon l'invention, on peut utiliser la méthylisobutylcétone (MIBC) à titre de solvant. Ce solvant est d'autant plus avantageux que le HMF est généralement issu de synthèse dissous dans la MIBC. Selon l'invention, on effectue alors la réaction avec une concentration de HMF de départ qui est supérieure à $6.10^{-2}$ moles par litre et une pression d'air supérieure à $5.10^5$ Pa, notamment de l'ordre de $10.10^5$ Pa.

Avantageusement et selon l'invention, on peut aussi utiliser le toluène à titre de solvant. Dans ce cas, selon l'invention, on effectue la réaction avec une concentration de HMF de départ comprise entre $1,10^{-2}$ et $8.10^{-2}$ moles par

litre, et une pression d'air supérieure à $10.10^5$ Pa, notamment de l'ordre de $16.10^5$ Pa.

Dans les exemples qui suivent, les réactions ont été effectuées dans un réacteur fermé de 100 ml muni d'un agitateur rotatif interne à entraînement magnétique à turbine, équipé d'une vanne d'arrivée de gaz sous pression et d'une vanne de prélèvement, chauffé, et équipé d'une régulation de température.

Le HMF utilisé, d'une pureté supérieure à 99 %, est un solide jaune clair, de masse molaire égale à 126, 11 g, qui fond à 35° C. Le FDC est un solide jaune paille, de masse molaire égale à 124 g et qui présente une longueur d'onde d'absorption en ultra-violets de 280 nm.

La quantité prédéterminée de HMF est préalablement dissoute dans 50 ml du solvant choisi, puis introduite avec le catalyseur solide dans le réacteur. Après une période d'agitation, on effectue un prélèvement permettant de doser précisément la quantité initiale de HMF dans le réacteur. On chauffe ensuite le réacteur sous agitation à 1 000 tr/min jusqu'à la température de réaction choisie, le temps de réaction étant compté à partir de l'obtention de cette température.

Les dosages du HMF et du FDC sont effectués par chromatographie HPLC à 15 min, 30 min, 60 min, 90 min, 150 min, 240 min de durée de réaction.

Lorsque le solvant utilisé est le toluène, la colonne de chromatographie est une colonne monosaccharide $H^+$. L'étalon utilisé est le furfural et le solvant d'élution est une solution aqueuse d'acide trifluoroacétique.

Lorsque le solvant utilisé est la MIBC, la colonne de chromatographie est une colonne contenant un support greffé CN. L'étalon utilisé est l'hydroquinone et le solvant d'élution est un mélange de cyclohexane (80 %), de dichlorométhane (16 %) et d'isopropanol (4 %).

EXEMPLE 1 :

Dans cet exemple, on utilise de l'oxyde de vanadium $V_2O_5$ massique (non supporté) avec un rapport massique du composé solide $V_2O_5$ sur le HMF introduit dans le réacteur égal à 2. Il est à noter que la proportion pondérale de vanadium dans $V_2O_5$ est de 56. Ce catalyseur est préalablement calciné pendant 4 heures à 500° C. La pression d'air est de $10.10^5$ Pa, la température est de 170° C et le solvant est le toluène.

On a tout d'abord testé la stabilité thermique du HMF à 170° C en l'introduisant sans catalyseur dissous dans le toluène. Au bout de 90 minutes, on constate une perte de 10 % du HMF.

En présence de catalyseur, les sélectivités obtenues sont de l'ordre de 70 % avec une conversion supérieure à 90 %. Après 90 min de réaction, la conversion est de 91 %, la sélectivité est de 69 %, soit un rendement de 62 %.

EXEMPLE 2 :

On réalise le même essai que dans l'exemple 1 en remplaçant l'oxyde de vanadium libre par de l'oxyde de vanadium supporté sur un support d'oxyde de titane en phase anatase.

Pour préparer ce catalyseur, 5 g d'oxyde de titane anatase sont mouillés avec de l'eau distillée puis étuvés pendant 20 h à 100° C. 560 mg de $NH_4VO_3$ sont ajoutés à une solution de 20 ml d'acide oxalique 1M. Le mélange est chauffé jusqu'à obtention d'une couleur bleu nuit et la solution est additionnée au support. On maintient l'agitation 30 min, puis on évapore, et on sèche à l'étuve à 100° C. Le catalyseur est ensuite broyé, tamisé et calciné 5 h à 450° C.

L'analyse du catalyseur obtenu démontre qu'il comprend 8,17 % en poids de vanadium (analyse centésimale) et a une surface spécifique de 9 $m^2$/g (méthode Brunauer-Emett-Teller, isothermes d'adsorption d'azote).

Les sélectivités obtenues sont comprises entre 60 et 70 % avec une conversion qui est supérieure à 90 % après seulement 30 min de réaction. La cinétique de transformation est donc plus rapide. Au bout de 90 min, la conversion est de 95 %, la sélectivité est de 67 %, soit un rendement de 64 %.

EXEMPLE 3 :

On prépare tout d'abord des composés solides formés d'oxyde de vanadium supporté sur oxyde de titane comprenant 64 % de phase anatase et 36 % de phase rutile.

10 g de ce support $TiO_2$ comprenant 64 % de phase anatase et 36 % de phase rutile (désigné par la suite $TiO_2$(A/R), partiellement déshydroxylé (TiOH) sont mis en solution dans 250 ml d'eau en présence d'une quantité variant entre 0,23 g et 4,56 g de métavanadate d'ammonium ($NH_4VO_3$). On acidifie la solution par ajout d'acide chlorhydrique concentré jusqu'à pH 2. Le mélange est agité 24 heures, puis centrifugé et le surnageant est éliminé.

Le précipité récupéré est lavé plusieurs fois à l'eau chaude (70° C - 100° C) pour éliminer les espèces excédentaires qui n'ont pas réagi. Après chaque lavage, le mélange est centrifugé et le surnageant est éliminé.

On récupère ensuite le catalyseur, on le sèche à l'étuve (60° C), on le broye, on le tamise (diamètre compris entre 0,063 mm et 0,125 mm), et on le calcine sous un flux d'air à 500° C pendant 4 heures.

En faisant varier la quantité de $NH_4VO_3$ de départ, on a obtenu des catalyseurs C1 à C6 comprenant des proportions différentes d'oxyde de vanadium. L'analyse centésimale a permis de doser la quantité de vanadium du catalyseur et de phase active $V_2O_5$ par rapport au support $TiO_2$.

La surface spécifique des catalyseurs a aussi été déterminée par la méthode Brunauer-Emett-Teller des isothermes d'adsorption d'azote.

| Catalyseur | % vanadium dosé (g V/100 g catalyseur) | % vanadium dosé (g $V_2O_5$/100 g $TiO_2$) | Surface spécifique BET m$^2$/g |
|---|---|---|---|
| $TiO_2$(A/R) | 0 | 0 | 72 |
| C1 | 1,32 | 2,40 | 53 |
| C2 | 1,67 | 3,07 | 50 |
| C3 | 4,79 | 9,34 | 44 |
| C4 | 7,12 | 14,55 | 39 |
| C5 | 7,33 | 15,03 | 42 |
| C6 | 8,6 | 18,20 | 38 |

Dans la suite, les différents catalyseurs $V_2O_5$ sur $TiO_2$(A/R) sont désignés par la référence (C1 à C6) donnée dans le tableau ci-dessus.

Le même essai que dans l'exemple 1 (170° C) a été réalisé en remplaçant le $V_2O_5$ non supporté par le catalyseur C5 ($V_2O_5$ sur $TiO_2$(A/R) à 7,33 % de vanadium).

Les sélectivités obtenues sont comprises entre 70 % et 80 % avec une conversion qui atteint 80 % dès que la durée de réaction est supérieure ou égale à 90 min. Pour une durée de 90 min, la conversion est de 80 % et la sélectivité est de 77 %.

EXEMPLE 4 :

Le même essai que dans l'exemple 3 a été réalisé mais à une température de 90° C, sous une pression d'air de 16.10$^5$ Pa et avec les catalyseurs C5 (7,33 % vanadium), $TiO_2$(A/R) seul (0 % vanadium), $V_2O_5$ non supporté (56 % vanadium), et $V_2O_5$ sur $TiO_2$ 100 % anatase (8,17 % vanadium).

Avec $V_2O_5$ non supporté, les sélectivités obtenues sont comprises entre 75 % et 80 % mais la conversion reste inférieure à 30 %. Le rendement reste inférieur à 25 % à cette température.

Avec le support $TiO_2$(A/R) seul, les sélectivités obtenues sont supérieures à 94 % mais avec une conversion inférieure à 15 %. Le rendement était donc inférieur à 14 %.

Avec $V_2O_5$ sur $TiO_2$ 100 % anatase, les sélectivités obtenues ont varié entre 75 % et 90 % avec une conversion inférieure à 40 %. Le rendement maximum obtenu après 240 min était de 31 %.

Avec $V_2O_5$ sur $TiO_2$(A/R), les sélectivités et les conversions obtenues, donc les rendements, sont nettement supérieurs. Ainsi, le rendement est supérieur à 66 % dès 60 min de réaction. Après 90 min, la conversion était de 81 % pour une sélectivité de 97 %, soit un rendement de 79 %. Les sélectivités étaient supérieures à 80 % avec des conversions supérieures à 54 % dès 30 min de réaction.

On constate ainsi, à 90° C, un effet de synergie entre $V_2O_5$ et le support $TiO_2$(A/R) (64 % anatase et 36 % rutile).

EXEMPLE 5 :

On a ensuite comparé les résultats obtenus avec les autres catalyseurs $V_2O_5$ / $TiO_2$(A/R) de proportions pondérales de vanadium différentes. Les essais ont été effectués dans les conditions de l'exemple 4 dans le toluène puis dans la MIBC, avec un rapport massique du catalyseur sur le HMF de 2 (0,4 g de catalyseur pour 0,2 g de HMF dans 50 ml de solvant). On a aussi déterminé les conversions et les sélectivités, donc les rendements pour des temps de réaction de 1 et 4 heures.

| TOLUENE, 90° C, 16.10$^5$ Pa d'air | | | | | |
|---|---|---|---|---|---|
| Catalyseur | % de V | Conversion en % à 1 h de réaction | Sélectivité en % à 1 h de réaction | Conversion en % à 4 h de réaction | Sélectivité en % à 4 h de réaction |
| - | 0 | 8 | 90 | 14 | 92 |
| C1 | 1,32 | 73 | 87 | 97 | 85 |
| C2 | 1,67 | 56 | 90 | 94 | 92 |
| C3 | 4,79 | 57 | 97 | 93 | 96 |
| C4 | 7,12 | 76 | 95 | 97 | 96 |
| C5 | 7,33 | 76 | 87 | 91 | 93 |
| C6 | 8,6 | 81 | 70 | 100 | 78 |

**MIBC, 90° C, 16.10$^5$ Pa d'air**

| Catalyseur | % de V | Conversion en % à 1 h de réaction | Sélectivité en % à 1 h de réaction | Conversion en % à 4 h de réaction | Sélectivité en % à 4 h de réaction |
|---|---|---|---|---|---|
| C1 | 1,32 | 31 | 90 | 79 | 78 |
| C3 | 4,79 | 42 | 90 | 93 | 70 |
| C4 | 7,12 | 47 | 92 | 91 | 80 |
| C5 | 7,33 | 26 | 97 | 66 | 98 |
| C6 | 8,6 | 44 | 82 | 91 | 77 |

En traçant les courbes correspondant à ces tableaux, on démontre que la monocouche est atteinte avec une proportion de vanadium supérieure à 4 %. En conséquence, la proportion de vanadium est avantageusement supérieure à 4 %.

EXEMPLE 6 :

Avec le catalyseur C5 (7,33 % de vanadium) on a évalué l'influence de la masse de catalyseur introduite sur la conversion du HMF, la sélectivité en FDC, dans le toluène puis dans la MIBC, dans les conditions de l'exemple 4.

**TOLUENE, 90° C, 16.10⁵ Pa d'air**

| Masse de catalyseur (g) | g catalyseur/ g HMF (%) | Conversion en % à 1 h de réaction | Sélectivité en % à 1 h de réaction | Conversion en % à 4 h de réaction | Sélectivité en % à 4 h de réaction |
|---|---|---|---|---|---|
| 0,1 | 0,5 | 38 | 87 | 82 | 82 |
| 0,4 | 2 | 81 | 70 | 100 | 78 |
| 0,6 | 3 | 78 | 87 | 100 | 85 |
| 0,8 | 4 | 96 | 85 | 100 | 78 |

**MIBC, 90° C, 16.10⁵ Pa d'air**

| Masse de catalyseur (g) | g catalyseur/ g HMF (%) | Conversion en % à 1 h de réaction | Sélectivité en % à 1 h de réaction | Conversion en % à 4 h de réaction | Sélectivité en % à 4 h de réaction |
|---|---|---|---|---|---|
| 0,4 | 2 | 44 | 82 | 91 | 77 |
| 0,6 | 3 | 50 | 85 | 96 | 67 |
| 0,8 | 4 | 64 | 80 | 100 | 50 |

EXEMPLE 7 :

Avec 0,4 g de catalyseur C5 (7,33 % vanadium), on a évalué l'influence de la quantité initiale de HMF sur la conversion et la sélectivité en FDC, dans le toluène et la MIBC, dans 50 ml, dans les conditions de l'exemple 4.

**TOLUENE, 90° C, 16.10$^5$ Pa d'air**

| Masse HMF (g) | g catalyseur/ g HMF (%) | Conversion en % à 1 h de réaction | Sélectivité en % à 1 h de réaction | Conversion en % à 4 h de réaction | Sélectivité en % à 4 h de réaction |
|---|---|---|---|---|---|
| 0,1 | 4 | 92 | 97 | 97 | 97 |
| 0,2 | 2 | 76 | 87 | 91 | 93 |
| 0,4 | 1 | 48 | 66 | 67 | 85 |
| 0,6 | 0,67 | 31 | 92 | 57 | 87 |
| 1,2 | 0,33 | 44 | 32 | 74 | 22 |

**MIBC, 90° C, 16.10$^5$ Pa d'air**

| Masse HMF (g) | g catalyseur/ g HMF (%) | Conversion en % à 1 h de réaction | Sélectivité en % à 1 h de réaction | Conversion en % à 4 h de réaction | Sélectivité en % à 4 h de réaction |
|---|---|---|---|---|---|
| 0,2 | 2 | 26 | 97 | 66 | 99 |
| 0,4 | 1 | 24 | 98 | 68 | 70 |
| 0,6 | 0,67 | 25 | 61 | 54 | 74 |
| 1,2 | 0,33 | 28 | 93 | 84 | 62 |

EXEMPLE 8 :

L'influence de la pression d'air a été évaluée avec le catalyseur C5 ($V_2O_5$ sur $TiO_2$(A/R) à 7,33 % de vanadium) à 90° C, le rapport massique du catalyseur sur le substrat étant de 2 (0,4 g de catalyseur pour 0,2 g de HMF), dans le toluène, puis dans la MIBC. On a fait varier la pression d'air de 0 à 26.10$^5$ Pa.

| Pression d'air ($10^5$ Pa) | Conversion en % à 1 h de réaction | Sélectivité en % à 1 h de réaction | Conversion en % à 4 h de réaction | Sélectivité en % à 4 h de réaction |
|---|---|---|---|---|
| TOLUENE | | | | |
| 0 | 15 | 95 | 25 | 95 |
| 5 | 62 | 85 | 92 | 83 |
| 10 | 71 | 81 | 96 | 78 |
| 16 | 76 | 87 | 91 | 93 |
| 26 | 79 | 85 | 98 | 80 |
| MIBC | | | | |
| 0 | 6 | 95 | 13 | 94 |
| 6 | 30 | 89 | 67 | 87 |
| 10 | 34 | 77 | 79 | 74 |
| 16 | 44 | 82 | 91 | 77 |
| 26 | 35 | 80 | 85 | 72 |

EXEMPLE 9 :

On a étudié l'influence de la température entre 75° C et 110° C, dans les mêmes conditions que l'exemple 8 en fixant la pression d'air à $16.10^5$ Pa.

| TOLUENE | Toluène 75° C | Toluène 90° C | Toluène 110° C | MIBC 75° C | MIBC 90° C | MIBC 110° C |
|---|---|---|---|---|---|---|
| Sélectivités obtenues | 75 % à 85 % | 80 % à 100 % | 90 % à 100 % | 80 % à 95 % | 85 % à 100 % | 40 % à 80 % |
| Conversion à 30 min | 34 | 54 | 86 | 15 | 12 | 52 |
| Conversion à 90 min | 49 | 81 | 97 | 28 | 40 | 84 |
| Sélectivité à 90 min | 83 | 98 | 100 | 85 | 89 | 70 |
| Rendement à 90 min | 41 | 79 | 97 | 24 | 36 | 59 |

Il ressort de ces essais que la réaction d'oxydation du HMF en FDC en milieu liquide présente une sélectivité et un rendement satisfaisants à basse température (moins de 200° C) en présence d'oxyde de vanadium. En particulier l'oxyde de vanadium supporté sur oxyde de titane comprenant entre 60 % et 75 % (notamment 64 %) de phase anatase et entre 40 % et 25 % (notamment 36 %) de phase rutile, procure à 90° C un effet synergique entre la phase active et le support (exemple 4), une sélectivité de 97 % pour un rendement de 79 % étant obtenue en 90 min.

En faisant varier la proportion pondérale de vanadium entre 1,19 % et 8,6 %, le rendement est toujours supérieur à 70 % et la sélectivité toujours supérieure à 78 % à 90° C (exemple 5). Entre 1,67 % et 7,33 %, le rendement reste supérieur à 84 % et la sélectivité reste supérieure à 92 % dans le toluène. A 8,6 % de vanadium, la conversion est totale et la sélectivité de 78 %.

Les exemples 6 et 7 montrent que le rapport massique du catalyseur sur le HMF peut varier entre environ 0,5 et 5 tout en procurant de bons résultats, les meilleurs résultats étant obtenus pour un rapport de 2.

Lorsque la concentration initiale de HMF est supérieure à $3,14.10^{-3}$ moles pour 50 ml, soit environ $6.10^{-2}$ moles/l, il est préférable d'utiliser la MIBC (exemple 7). Au contraire, si cette concentration est inférieure à $3,88.10^{-3}$ moles pour 50 ml, soit environ $8.10^{-2}$ moles/l, il est préférable d'utiliser le toluène.

En outre, l'exemple 8 montre que la conversion ne change pratiquement plus (75-80 %) dès $16.10^5$ Pa d'air

dans le toluène. Dans la MIBC, la conversion maximale (35-40 %) est atteinte dès $10.10^5$ Pa d'air.

Enfin, l'exemple 9 montre qu'à 75° C la conversion n'est pas très importante, qu'à 90° C la conversion et la sélectivité sont bonnes, et qu'à 110° C la sélectivité dans la MIBC baisse.

A partir de ces résultats, on peut envisager la production industrielle en continu du FDC, par exemple avec un ou plusieurs étages de réacteur multicontact notamment du type colonne pulsée, à partir du HMF en phase liquide à 90° C en présence de $V_2O_5$, notamment sous forme supporté sur $TiO_2$ comprenant 64 % de phase anatase et 36 % de phase rutile à 7,33 % de vanadium.

Parmi les autres oxydes ou sels métalliques pouvant être utilisés, on peut citer notamment les oxydes ou sels de titane, de zirconium, de vanadium, de niobium, de chrome, de molybdène et de tungstène. Les oxydes ou sels métalliques peuvent être utilisés à l'état massique (non supporté) ou au contraire à l'état supporté.

**Revendications**

1.  Procédé de fabrication de furanedicarboxaldéhyde-2,5 (FDC) à partir de l'hydroxyméthyl-5 furane carboxaldéhyde-2 (HMF) en milieu liquide, caractérisé en ce qu'on met le HMF dissous dans un solvant liquide au contact d'au moins un composé solide comprenant au moins un oxyde de métal choisi parmi les métaux des colonnes IV B, V B et VI B de la classification périodique des éléments, et on place le milieu réactionnel à une température comprise entre 75° C et 200° C.

2.  Procédé selon la revendication 1, caractérisé en ce qu'on utilise un composé solide comprenant de l'oxyde de vanadium.

3.  Procédé selon la revendication 2, caractérisé en ce qu'on utilise un composé solide qui est constitué d'oxyde de vanadium non supporté.

4.  Procédé selon la revendication 2, caractérisé en ce qu'on utilise, à titre de composé solide, de l'oxyde de vanadium supporté sur un support d'oxyde métallique de type amphotère à prédominance acide.

5.  Procédé selon la revendication 4, caractérisé en ce qu'on utilise de l'oxyde de vanadium supporté sur un support d'oxyde de titane.

6.  Procédé selon la revendication 5, caractérisé en ce que le support d'oxyde de titane est formé d'oxyde de titane comprenant entre 50 % et 100 % de phase anatase et entre 50 % et 0 % de phase rutile.

7.  Procédé selon la revendication 6, caractérisé en ce que le support d'oxyde de titane est formé d'oxyde de titane comprenant entre 60 % et 75 % de phase anatase et entre 40 % et 25 % de phase rutile.

8.  Procédé selon la revendication 7, caractérisé en ce que le support d'oxyde de titane est formé d'oxyde de titane comprenant de l'ordre de 64 % de phase anatase et de l'ordre de 36 % de phase rutile.

9.  Procédé selon l'une des revendications 6 à 8, caractérisé en ce qu'on effectue la réaction à une température inférieure à 100° C, notamment de l'ordre de 90° C.

10. Procédé selon l'une des revendications 2 à 9, caractérisé en ce qu'on utilise un composé solide comprenant entre 1 % et 56 % en poids de vanadium.

11. Procédé selon la revendication 10, caractérisé en ce qu'on utilise un composé solide comprenant au moins 4 % en poids de vanadium.

12. Procédé selon l'une des revendications 1 à 11, caractérisé en ce qu'on utilise le composé solide selon un rapport massique par rapport à la quantité initiale de HMF compris entre 0,5 et 5, notamment de l'ordre de 2.

13. Procédé selon l'une des revendications 2 à 12, caractérisé en ce qu'on effectue la réaction en présence d'oxygène sous une pression partielle suffisante pour maintenir l'oxyde de vanadium à son niveau d'oxydation optimum pour la réaction.

14. Procédé selon la revendication 13, caractérisé en ce qu'on effectue la réaction dans un réacteur dans lequel on maintient une pression de gaz comprenant de l'oxygène.

15. Procédé selon la revendication 14, caractérisé en ce qu'on effectue la réaction dans un réacteur dans lequel on maintient une pression d'air comprise entre $5.10^5$ Pa et $30.10^5$ Pa.

16. Procédé selon l'une des revendications 1 à 15, caractérisé en ce qu'on place le HMF dans un solvant polaire liquide apte à solubiliser le HMF et à rester chimiquement inerte à la température de réaction et vis-à-vis du composé solide.

17. Procédé selon la revendication 16, caractérisé en ce que le solvant est la méthylisobutylcétone (MIBC).

18. Procédé selon la revendication 17, caractérisé en ce qu'on effectue la réaction avec une concentration de HMF de départ supérieure à $6.10^{-2}$ moles par litre et une pression d'air supérieure à $5.10^5$ Pa, notamment de l'ordre de $10.10^5$ Pa.

19. Procédé selon la revendication 16, caractérisé en ce que le solvant est le toluène.

20. Procédé selon la revendication 19, caractérisé en ce qu'on effectue la réaction avec une concentration de HMF de départ comprise entre $1.10^{-2}$ et $8.10^{-2}$ moles par litre et une pression d'air supérieure à $10.10^5$ Pa, notamment de l'ordre de $16.10^5$ Pa.

**Patentansprüche**

1. Verfahren zur selektiven Herstellung von 2,5-Furandicarboxaldehyd (FDC) aus 5-Hydroxymethyl-2-furancarboxaldehyd (HMF) in der Flüssigphase, dadurch gekennzeichnet, dass man das in einem flüssigen Lösungsmittel gelöste HMF mit mindestens einem Feststoff in Kontakt bringt, der mindestens ein Metalloxyd enthält dessen Metall aus den Spalten IV B, V B und/oder VI B des Periodensystems der Elemente gewählt ist, und dass man die Reaktionsumgebung auf eine Temperatur zwischen 75°C und 200°C bringt.

2. Verfahren nach Anspruch 1, dadurch gekennzeichnet, dass man einen Feststoff verwendet der Vanadiumoxyd enthält.

3. Verfahren nach Anspruch 2, dadurch gekennzeichnet, dass man einen Feststoff verwendet der aus Vanadiumoxyd ohne Trägermaterial besteht.

4. Verfahren nach Anspruch 2, dadurch gekennzeichnet, dass man als Feststoff Vanadiumoxyd verwendet, das auf ein Trägermaterial aus einem amphoteren, überwiegend sauren Metalloxyd aufgebracht ist.

5. Verfahren nach Anspruch 4, dadurch gekennzeichnet, dass man auf Titanoxyd als Trägermaterial aufgebrachtes Vanadiumoxyd verwendet.

6. Verfahren nach Anspruch 5, dadurch gekennzeichnet, dass das Trägermaterial aus Titanoxyd zu 50% bis 100% in Form von Anatas und zu 50% bis 0% in Form von Rutil vorliegt.

7. Verfahren nach Anspruch 6, dadurch gekennzeichnet, dass das Trägermaterial aus Titanoxyd zu 60% bis 75% in Form von Anatas und zu 40% bis 25% in Form von Rutil vorliegt.

8. Verfahren nach Anspruch 7, dadurch gekennzeichnet, dass das Trägermaterial aus Titanoxyd zu ungefähr 64% in Form von Anatas und zu ungefähr 36% in Form von Rutil vorliegt.

9. Verfahren nach irgendeinem der Ansprüche 6 bis 8, dadurch gekennzeichnet, dass die Reaktion bei einer Temperatur unter 100°C durchgeführt wird, und zwar insbesondere bei ungefähr 90°C.

10. Verfahren nach irgendeinem der Ansprüche 2 bis 9, dadurch gekennzeichnet, dass man einen Feststoff verwendet, der zwischen 1% und 56% (Massenprozent) Vanadium enthält.

11. Verfahren nach Anspruch 10, dadurch gekennzeichnet, dass man einen Feststoff verwendet der mindestens 4% (Massenprozent) Vanadium enthält.

12. Verfahren nach irgendeinem der Ansprüche 1 bis 11, dadurch gekennzeichnet, dass man den Feststoff in einem Massenverhältnis von 0,5 bis 5, insbesondere einem Massenverhältnis von ungefähr 2, bezogen auf die anfängliche Menge von HMF verwendet.

13. Verfahren nach irgendeinem der Ansprüche 2 bis 12, dadurch gekennzeichnet, dass man die Reaktion in Gegenwart von Sauerstoff durchführt, mit genügendem Sauerstoffpartialdruck um das Vanadiumoxyd auf seiner für die Reaktion optimalen Oxydationsstufe zu halten.

14. Verfahren nach Anspruch 13, dadurch gekennzeichnet, dass man die Reaktion in einem Reaktor durchführt, in dem man ein sauerstoffhaltiges Gas unter Druck hält.

15. Verfahren nach Anspruch 14, dadurch gekennzeichnet, dass man die Reaktion in einem Reaktor durchführt, in dem man einen Luftdruck zwischen $5.10^5$ Pa und $30.10^5$ Pa aufrechterhält.

16. Verfahren nach irgendeinem der Ansprüche 1 bis 15, dadurch gekennzeichnet, dass man das HMF in einem polaren, flüssigen Lösungsmittel löst, das geeignet ist, das HMF zu lösen und bei der Reaktionstemperatur nicht chemisch mit dem Feststoff zu reagieren.

17. Verfahren nach Anspruch 16, dadurch gekennzeichnet, dass das Lösungsmittel Methylisobutylketon (MIBK) ist.

18. Verfahren nach Anspruch 17, dadurch gekennzeichnet, dass man die Reaktion mit einer anfänglichen HMF-Konzentration größer als $6.10^{-2}$ Mol pro Liter, und einem Luftdruck von über $5.10^5$ Pa, insbesondere von ungefähr $10.10^5$ Pa durchführt.

19. Verfahren nach Anspruch 16, dadurch gekennzeichnet, dass das Lösungsmittel Toluol ist.

20. Verfahren nach Anspruch 19, dadurch gekennzeichnet, dass man die Reaktion mit einer anfänglichen HMF-Konzentration zwischen $1.10^{-2}$ Mol pro Liter und $8.10^{-2}$ Mol pro Liter und einem Luftdruck von über $10.10^5$ Pa, insbesondere von ungefähr $16.10^5$ Pa durchführt.

**Claims**

1. A process for the manufacture of furan-2,5-dicarboxaldehyde (FDC) from 5-hydroxymethylfuran-2-carboxaldehyde (HMF) in liquid medium, characterized in that the HMF dissolved in a liquid solvent is placed in contact with at least one solid compound comprising at least one oxide of a metal chosen from the metals of columns IVB, VB and VIB of the Periodic Table of the Elements, and the reaction medium is placed at a temperature of between 75°C and 200°C.

2. A process according to claim 1, characterized in that a solid compound comprising vanadium oxide is used.

3. A process according to claim 2, characterized in that a solid compound which consists of unsupported vanadium oxide is used.

4. A process according to claim 2, characterized in that vanadium oxide supported on a support of metal oxide of predominantly acidic amphoteric type is used as solid compound.

5. A process according to claim 4, characterized in that vanadium oxide supported on a titanium oxide support is used.

6. A process according to claim 5, characterized in that the titanium oxide support is formed of titanium oxide comprising between 50% and 100% anatase phase and between 50% and 0% rutile phase.

7. A process according to claim 6, characterized in that the titanium oxide support is formed of titanium oxide comprising between 60% and 75% anatase phase and between 40% and 25% rutile phase.

8. A process according to claim 7, characterized in that the titanium oxide support is formed of titanium oxide comprising about 64% anatase phase and about 36% rutile phase.

9.  A process according to one of claims 6 to 8, characterized in that the reaction is carried out at a temperature of below 100°C, in particular of about 90°C.

10. A process according to one of claims 2 to 9, characterized in that a solid compound comprising between 1% and 56% by weight of vanadium is used.

11. A process according to claim 10, characterized in that a solid compound comprising at least 4% by weight of vanadium is used.

12. A process according to one of claims 1 to 11, characterized in that the solid compound is used in a mass ratio relative to the initial amount of HMF of between 0.5 and 5, in particular of about 2.

13. A process according to one of claims 2 to 12, characterized in that the reaction is carried out in the presence of oxygen under a partial pressure which is sufficient to maintain the vanadium oxide at its optimum level of oxidation for the reaction.

14. A process according to claim 13, characterized in that the reaction is carried out in a reactor in which a gas comprising oxygen is maintained at pressure.

15. A process according to claim 14, characterized in that the reaction is carried out in a reactor in which an air pressure of between $5 \times 10^5$ Pa and $30 \times 10^5$ Pa is maintained.

16. A process according to one of claims 1 to 15, characterized in that the HMF is placed in a liquid polar solvent capable of solubilizing the HMF and of remaining chemically inert at the reaction temperature and with respect to the solid compound.

17. A process according to claim 16, characterized in that the solvent is methyl isobutyl ketone (MIBK).

18. A process according to claim 17, characterized in that the reaction is carried out with a starting HMF concentration of greater than $6 \times 10^{-2}$ mol per liter and an air pressure of greater than $5 \times 10^5$ Pa, in particular of about $10 \times 10^5$ Pa.

19. A process according to claim 16, characterized in that the solvent is toluene.

20. A process according to claim 19, characterized in that the reaction is carried out with a starting HMF concentration of between $1 \times 10^{-2}$ and $8 \times 10^{-2}$ mol per liter and an air pressure greater than $10 \times 10^5$ Pa, in particular of about $16 \times 10^5$ Pa.